# EUROPEAN PATENT SPECIFICATION

(11) **EP 1 504 773 B1**
(45) Date of publication and mention of the grant of the patent: **28.10.2009**
(21) Application number: 04254785.1
(22) Date of filing: 09.08.2004
(51) Int. Cl.: A61L 17/14

(54) **High strength suture with collagen fibres**
Hoch-festes Nahtmaterial mit Kollagenfasern
Suture comprenant des fibres de collagène à haute resistance

(30) Priority: 07.08.2003 US 492972 P
(43) Date of publication of application: 09.02.2005
(73) Proprietor: Arthrex, Inc., Naples, FL 34108-1945 (US)
(72) Inventor: Grafton, Donald R., Naples 34112, Florida (US)
(74) Representative: Collins, John David

(56) References cited:
- EP-A- 0 561 108
- EP-A- 1 293 218
- EP-A- 1 300 115
- WO-A-01/56626
- DE-A- 2 552 097

## Description

### BACKGROUND OF THE INVENTION

### 1. Field of the Invention:

The present invention relates to high strength surgical suture materials, and more particularly to braided suture that includes fibers of long-chain, ultrahigh molecular weight polyethylene for strength and fibers of collagen for improved tissue remodeling and biocompatibility.

### 2. Description of the Related Art:

Suture strength and tissue compatibility are important considerations in any surgical suture material. One of the strongest materials currently formed into elongated strands is an ultrahigh molecular weight long chain polyethylene, typically used for fishing line and the like, which is sold under the trade names Dyneema or Spectra. This material is much stronger than ordinary surgical suture, however, it does not have acceptable knot tie down characteristics for use in surgical applications, and is not bioabsorbable.

Preparation of collagen fibers for use as surgical suture is known in the prior art. See U.S. Pat. Nos. 5,814,328 and 6,548,077 to Gunasekaran, 5,374,539 to Nimni et al., and 5,618,312 to Yui et al., for example.

A suture made of a high-strength braid of long-chain polyethylene and polyester developed by the assignee of the present application has acceptable tie down characteristics. See U.S. Patent No. 6,716,234.

It would be advantageous to have high-strength suture with improved tissue compatibility and enhanced tissue remodeling capabilities.

### SUMMARY OF THE INVENTION

The present invention provides a suture structure suitable for use as a suture or ligature comprising a plurality of first fibers comprised of ultrahigh molecular weight polyethylene, a plurality of second fibers comprised of at least one long chain synthetic polymer or bioabsorbable fiber, said second fibers not comprising fibers of ultrahigh molecular weight polyethylene, and a plurality of third fibers of a collagen, said first, second and third fibers being braided together to produce the suture strand.

The present invention advantageously provides a high strength surgical suture material with improved tissue compatibility. The suture can feature a braided cover made of a braid of ultrahigh molecular weight long chain polyethylene fiber and, optionally, a fiber of one or more long chain synthetic polymers, preferably polyester. Fibers of collagen can also be included in the braided cover. The polyethylene provides strength. The polyester, if included, improves tie down properties. The collagen enhances tissue remodeling and biocompatibility. Additionally, the collagen can form the base for adding growth factors to the suture, such as PDGF (platelet derived growth factor).

The preferred composition of the braided cover includes about 50% long-chain polyethylene, about 30% polyester, and about 20% collagen, preferably Type I bovine collagen. Filaments of Type I bovine collagen are provided by Kensey Nash Corporation. Preferably, about fifteen collagen filaments are braided together into ends. The ends are provided on bobbins, and are braided with the polyethylene and polyester into the braided suture cover.

Handling properties of the high strength suture, such as flexibility, hand, chatter, and drag, also can be enhanced using various materials to coat the suture. In addition, strands of a contrasting color can be added to the braided threads to enhance visibility. The colored strands preferably are dyed filaments of polyester or nylon. In an exemplary embodiment, half of a length of suture is provided with colored tracing strands, or otherwise contrasts with the other half of the length of suture, which remains a plain, solid color, for example. Accordingly, when the length of suture is loaded through the eyelet of a suture anchor, for example, identification and movement of the suture legs is simplified. The half-trace/half-plaia arrangement of the suture facilitates tracing and identification of the two legs of the suture during arthroscopic surgery.

In a preferred embodiment, the suture includes a multifilament cover formed of ultrahigh molecular weight polyethylene fiber braided with polyester and/or nylon fibers, and Type I bovine collagen fibers. The cover surrounds a fiber core made substantially or entirely of ultrahigh molecular weight polyethylene. The core preferably includes three strands of ultrahigh molecular weight polyethylene, twisted at about three to six twists per inch.

The braided cover preferably includes eight strands of ultrahigh molecular weight polyethylene braided with six strands of polyester and two Type I bovine collagen strands. In addition, strands of nylon can be provided in black or some other contrasting color as described in co-pending U.S. application Serial No. 10/358,399 incorporated herein by reference.

Ultrahigh molecular weight polyethylene fibers suitable for use in the present invention are marketed under the Dyneema trademark by Toyo Boseki Kabushiki Kaisha, and are produced in the U.S. by Honeywell International Inc., under the trademark Spectra.

The suture of an embodiment of the present invention advantageously has the strength of Ethibond #5 suture (Ethicon, Inc.), yet has the diameter, feel and tic ability of #2 suture, and promotes remodeling of surrounding tissue. As a result, the suture is ideal for most orthopedic procedures such as rotator cuff repair, Achilles tendon repair, patellar tendon repair, ACL/PCL reconstruction, hip and shoulder reconstruction procedures, and replacement for suture used in or with suture anchors.

The suture can be uncoated, or coated with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, or abrasion resistance, for example.

As an added advantage, as mentioned above, all or some of the nylon fibers (or the polyester fibers) in the cover may be provided in a contrasting color for visibility and identification purposes. A few trace threads having a contrasting color, preferably of a readily dyed fiber such as polyester or nylon, in the cover aid surgeons in identifying the travel direction of the suture during surgery, particularly during arthroscopic operations. Providing the trace threads in a regularly repeating pattern is particularly useful, allowing the surgeon to distinguish different ends of lengths of suture, and determine the direction of travel of a moving length of suture. The trace threads preferably are provided on only half of a length of suture to allow for tracing and identification of the two halves of the suture, such as when the suture is threaded through an eyelet of a suture anchor. Of the more easily dyed fibers, nylon is preferred in that it accepts dye readily. Polyester fibers are stronger, but do not take up dye as easily as nylon.

The present invention is not limited by the type of collagen fiber used or the method by which is it obtained. Collagen fibers used in the present invention can be produced, extracted, or harvested from animal tissue, and may be generated or modified by various processes, including enzymatic treatment of collagen material also including treatments using microorganisms. The result collagen products or intermediates can undergo separation and purification, and may be enzymatically or chemically modified to enhance various properties of the collagen-based material. It may also be possible to derive collagen fibers by way of synthetic methods for manufacturing polymer proteins or polypeptides. Moreover, as mentioned previously, growth factors, such as PDGF, can be added to the collagen strands.

The suture, including the cover and the core, and the strands making up the suture, can be formed from individual or multiple filaments or yarns by various known methods, including but not limited to being entangled, twisted, plied, braided, and knitted.

Other features and advantages of the present invention will become apparent from the following description of the invention which refers to the accompanying drawings.

### BRIEF DESCRIPTION OF THE DRAWINGS

Fig. 1 is a copy of a scanning electron micrograph of a length of suture according to one embodiment of the present invention.
Fig. 2 is a schematic cross section of a length of suture according to one embodiment of the present invention.
Fig. 3 is an illustration of the suture of one embodiment of the present invention attached to a suture anchor.
Fig. 4A and 4B show the suture of one embodiment of the present invention attached to a half round, tapered needle.
Fig. 5 illustrates a bulk length of suture of one embodiment of the present invention.
Fig. 6 illustrates a strand of suture according to one embodiment of the present invention provided on a suture anchor.

### DETAILED DESCRIPTION OF THE INVENTION

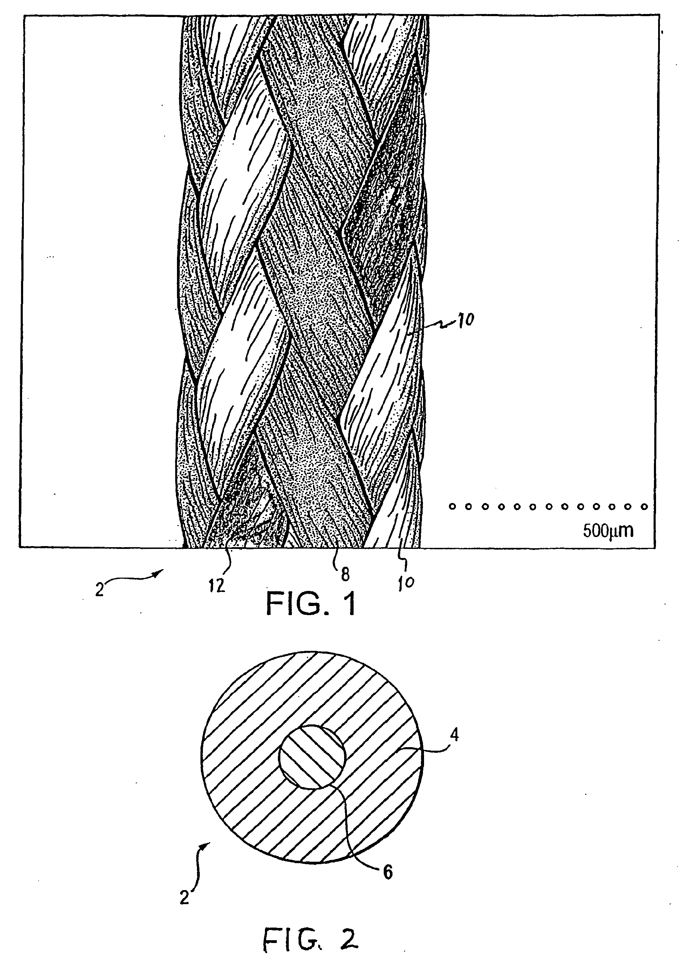

Referring to Fig. 1, a scanning electron micrograph of a length of suture 2 according to an embodiment of the present invention is shown. Suture 2 is made up of a cover 4 and a core 6 surrounded by the cover. See Fig. 2. Strands of long-chain, ultrahigh molecular weight polyethylene (UHMWPE) 8, sold under the tradename Spectra or Dyneema, strands of polyester 10 and strands of collagen 12 are braided together to form the cover 4. Core 6 is formed of twisted strands of UHMWPE.

UHMWPE strands 8 are substantially translucent or colorless. The majority of the polyester strands 10 are white (undyed). Some of the polyester or nylon strands 10 having a contrasting color can be included to provide a trace in the suture. Due to the transparent nature of the UHMWPE, the suture takes on the color of strands 10, and thus appears to be white, with a trace in the contrasting color.

Details of the present invention will be described further below in connection with the following examples:

### Example: USP Size 5 (EP size 7)

Made on a 16 carrier Hobourns machine, the yarns used in the braided cover are Honeywell Spectra 2000, polyester type 712, and Type I collagen. The cover is formed using eight strands of 144 decitex Spectra per carrier, braided with six strands of 100 decitex polyester, and two strands of collagen. The core is formed of three carriers of 144 decitex Spectra braided at three to six twists per inch. Alternatively, collagen fibers can be incorporated into the core as well.

The example set forth above is for size 5 suture. In the making of various sizes of the inventive suture, different decitex values and different PPI settings can be used to achieve the required size and strength needed. In addition, smaller sizes may require manufacture on 12 carrier machines, for example. The very smallest sizes preferably are made without a core. Overall, the suture may range from 5% to 90% ultrahigh molecular weight polymer (preferably at least 40% of the fibers are ultrahigh molecular weight polymer), with the balance formed of polyester and/or nylon, and collagen. Collagen preferably is present in amounts of about 20% of the braided cover. The core preferably comprises 18% or greater of the total amount of filament.

The suture preferably is coated with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, or abrasion resistance, for example.

The ultra high molecular weight (UHMW) polymer component of the present invention provides strength, and the polyester component is provided to improve tie ability and tie down characteristics. However, it has been found that the UHMW polymer provides an unexpected advantage of acting as a cushion for the polyester fibers, which are relatively hard and tend to damage each other. The UHMW polymer prevents breakage by reducing damage to the polyester when the suture is subjected to stress.

According to an alternative embodiment of the present invention, bioabsorbable suture is provided by braiding a high strength material, such as UHMWPE fibers, with a bioabsorbable material, such as PLLA or one of the other polylactides, for example, in addition to the collagen. Accordingly, a suture made with about 10% Spectra or Dyneema braided with absorbable fibers would provide greater strength than existing bioabsorbable suture with less stretch. Over time, 90% or more of the suture would absorb, leaving only a very small remnant of the knot. The absorbable suture can include coatings and colored traces as noted above for suture containing collagen as the only absorbable component.

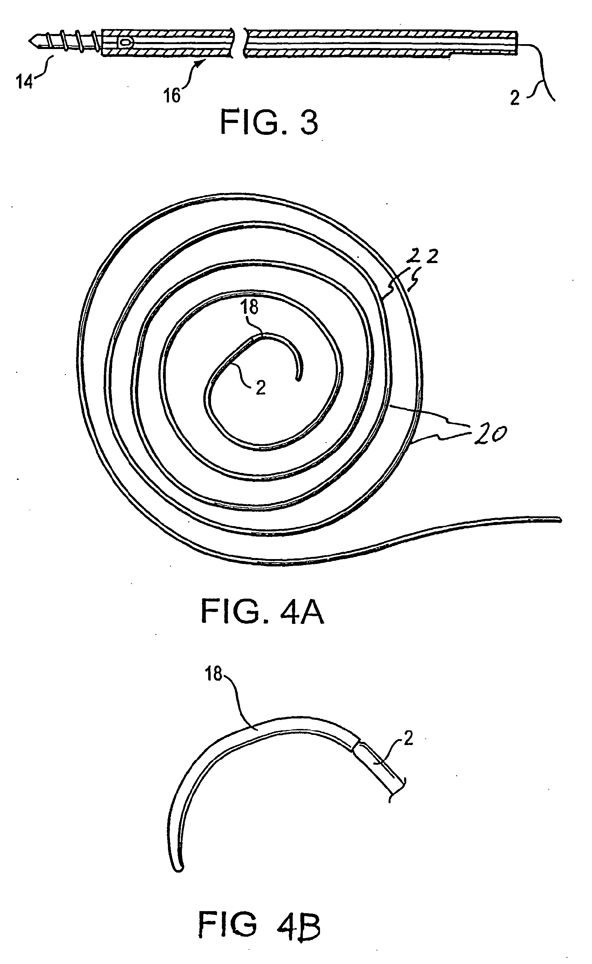

In one method of using the suture the suture 2 is attached to a suture anchor 14 as shown in Fig. 3 (prepackaged sterile with an inserter 16), or is attached at one or both ends to a half round, tapered needle 18 as shown in Figs. 4A and 4B. Fig. 4A also illustrates a length of suture having regularly repeating pattern of trace threads according to the present invention. Sections 20 of the length of suture 2 have tracing threads woven in, where sections 22 of the length of suture are plain, or otherwise are distinguishable from sections 20. The alternating patterned and plain sections aid the surgeon in determining the direction of suture travel, for example.

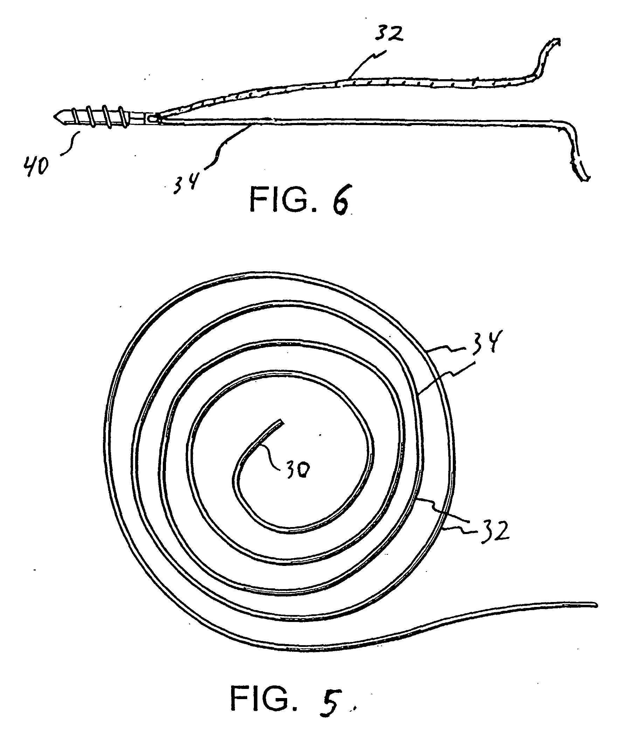

As shown in Fig. 5, bulk suture 30 is provided with repeating sections 32 having trace threads separated by sections 34 having no trace threads. The bulk suture is cut between every other section, at one end of each plain section, for example, to provide lengths of suture that are half traced and half plain. Alternatively, the bulk suture can be cut midway through each section to provide a shorter suture having a trace at one end. The half-and-half lengths of suture can be threaded through the eyelet of a suture anchor 40, as shown in Fig. 6. Accordingly, each leg of the suture strand provided on the suture anchor is easily distinguishable by a surgeon operating with the suture anchor assembly.

By providing strands of collagen, or other bioabsorbable polypeptides, in the suture, tissue remodeling is enhanced. Accordingly, as the collagen or other polpyeptide degrades, it is replaced by newly formed tissue generated by that surrounding the suture. As a result, remodeling of the tissue surrounding the repair site is enhanced. Additionally, the collagen strands can serve as a base for the addition of growth factor, such as PDGF, to the suture.

## Claims

1. A suture structure suitable for use as a suture or ligature comprising a plurality of first fibers comprised of ultrahigh molecular weight polyethylene, a plurality of second fibers comprised of at least one long chain synthetic polymer or bioabsorbable fiber, said second fibers not comprising fibers of ultrahigh molecular weight polyethylene, and a plurality of third fibers comprised of a collagen, said first, second and third fibers being braided together to produce the suture strand.

2. A suture strand as recited in claim 1, wherein sections of at least one of the plurality of fibers is provided in a color contrasting from the other fibers to provide an identifiable trace along a portion of the suture strand.

3. A suture strand as recited in claim 2, wherein at least one portion of said suture strand is provided with no identifiable trace.

4. A suture strand as recited in claim 1, wherein said at least one long chain synthetic polymer is polyester, nylon, or both.

5. A suture strand as recited in claim 1, wherein said ultrahigh molecular weight polyethylene comprises at least 40% of the braided fibers.

6. A suture strand as recited in claim 1, wherein said polyester comprises less than 40% of said braided filaments.

7. A suture strand as recited in claim 1, wherein said collagen comprises about 20% of said braided filaments.

8. A suture strand as recited in claim 1, further comprising a core of twisted fibers of ultrahigh molecular weight polyethylene surrounded by a cover comprising said first, second and third fibers.

9. A suture strand as recited in claim 8, wherein said core comprises about 18% or greater of the total amount of filament in said suture strand.

10. A suture strand as recited in claim 8, wherein said core further includes fibers of collagen.

11. A suture strand as recited in claim 8, wherein said cover comprises less than about 82% of the total amount of filament in said suture strand.

12. A suture strand as recited in claim 8, further comprising a coating disposed on said cover.

13. A suture strand as recited in claim 12, wherein said coating is selected from the group consisting of wax, silicone, silicone rubbers, PTFE, PBA, and ethyl cellulose.

14. A suture strand as recited in claim 1, wherein a half length of said suture strand is provided with an identifiable trace, and the other half length of said suture strand contains no identifiable trace.

15. A suture assembly comprising:
a suture strand according to any preceding claim; and
a suture anchor attached to said suture strand.

16. A suture assembly comprising:
a suture strand according to any one of claims 1 to 14; and
a half round, tapered needle attached to one or both ends of said suture strand.

## Patentansprüche

1. Zur Verwendung als Naht oder Ligaturfaden geeignetes Nahtmaterial, das eine Vielzahl von ersten Fasern, die aus ultrahochmolekularem Polyethylen bestehen, eine Vielzahl von zweiten Fasern, die aus mindestens einem langkettigen synthetischen Polymer oder bioabsorbierbaren Faser bestehen, wobei die zweiten Fasern keine Fasern aus ultrahochmolekularem Polyethylen aufweisen, und eine Vielzahl von dritten Fasern aufweist, die aus Kollagen bestehen, wobei die ersten, zweiten und dritten Fasern miteinander verflochten werden, um den Nahtmaterialstrang zu erzeugen.

2. Nahtmaterialstrang nach Anspruch 1, wobei Abschnitte mindestens einer der Vielzahl von Fasern in einer Farbe bereitgestellt werden, die mit den anderen Fasern kontrastiert, um entlang einem Abschnitt des Nahtmaterialstrangs eine identifizierbare Spur bereitzustellen.

3. Nahtmaterialstrang nach Anspruch 2, wobei mindestens ein Abschnitt des Nahtmaterialstrangs ohne identifizierbare Spur bereitgestellt wird.

4. Nahtmaterialstrang nach Anspruch 1, wobei das mindestens eine langkettige synthetische Polymer Polyester, Nylon oder beides ist.

5. Nahtmaterialstrang nach Anspruch 1, wobei das ultrahochmolekulare Polyethylen mindestens 40% der verflochtenen Fasern bildet.

6. Nahtmaterialstrang nach Anspruch 1, wobei der Polyester weniger als 40% der verflochtenen Filamente bildet.

7. Nahtmaterialstrang nach Anspruch 1, wobei das Kollagen etwa 20% der verflochtenen Filamente bildet.

8. Nahtmaterialstrang nach Anspruch 1, der ferner einen Kern von gezwirnten Fasern aus ultrahochmolekularem Polyethylen aufweist, der von einer Hülle umgeben ist, die erste, zweite und dritte Fasern aufweist.

9. Nahtmaterialstrang nach Anspruch 8, wobei der Kern etwa 18% oder mehr der Gesamtfadenmenge in dem Nahtmaterialstrang bildet.

10. Nahtmaterialstrang nach Anspruch 8, wobei der Kern ferner Kollagenfasern enthält.

11. Nahtmaterialstrang nach Anspruch 8, wobei die Hülle weniger als etwa 82% der Gesamtfadenmenge in dem Nahtmaterialstrang bildet.

12. Nahtmaterialstrang nach Anspruch 8, der ferner eine auf der Hülle aufgebrachte Beschichtung aufweist.

13. Nahtmaterialstrang nach Anspruch 12, wobei die Beschichtung aus der Gruppe ausgewählt ist, die aus Wachs, Silicon, Siliconkautschuken, PTFE, PBA und Ethylcellulose besteht.

14. Nahtmaterialstrang nach Anspruch 1, wobei eine halbe Länge des Nahtmaterialstrangs mit einer identifizierbaren Spur versehen ist und die andere halbe Länge des Nahtmaterialstrangs keine identifizierbare Spur enthält.

15. Nahtsystem, das aufweist:
einen Nahtmaterialstrang nach einem der vorstehenden Ansprüche; und
eine an dem Nahtmaterialstrang befestigte Nahtverankerung.

16. Nahtsystem, das aufweist:
einen Nahtmaterialstrang nach einem der Ansprüche 1 bis 14; und
eine halbrunde, spitz zulaufende Nadel, die an einem oder beiden Enden des Nahtmaterialstrangs befestigt ist.

## Revendications

1. Structure de suture appropriée pour une utilisation en tant que suture ou ligature comprenant une pluralité de premières fibres composées d'un polyéthylène à poids moléculaire ultra-élevé, une pluralité de deuxièmes fibres composées d'au moins un polymère synthétique à longue chaîne ou une fibre bioabsorbable, lesdites deuxièmes fibres ne comprenant pas de fibres d'un polyéthylène à poids moléculaire ultra-élevé, et une pluralité de troisièmes fibres composées d'un collagène, lesdites premières, deuxièmes et troisièmes fibres étant tressées ensemble pour produire le brin de suture.

2. Brin de suture selon la revendication 1, dans lequel des sections d'au moins une de la pluralité de fibres sont prévues dans une couleur contrastant avec les autres fibres pour donner une trace identifiable le long d'une portion du brin de suture.

3. Brin de suture selon la revendication 2, dans lequel au moins une portion dudit brin de suture est prévue sans trace identifiable.

4. Brin de suture selon la revendication 1, dans lequel ledit au moins un polymère synthétique à longue chaîne est un polyester, un nylon ou les deux.

5. Brin de suture selon la revendication 1, dans lequel ledit polyéthylène à poids moléculaire ultra-élevé comprend au moins 40% des fibres tressées.

6. Brin de suture selon la revendication 1, dans lequel ledit polyester comprend moins de 40% desdits filaments tressés.

7. Brin de suture selon la revendication 1, dans lequel ledit collagène comprend environ 20% desdits filaments tressés.

8. Brin de suture selon la revendication 1, comprenant en outre une âme de fibres tordues d'un polyéthylène à poids moléculaire ultra-élevé entourée par une enveloppe comprenant lesdites premières, deuxièmes et troisièmes fibres.

9. Brin de suture selon la revendication 8, dans lequel ladite âme comprend environ 18% ou plus de la quantité totale de filament dans ledit brin de suture.

10. Brin de suture selon la revendication 8, dans lequel ladite âme inclut en outre des fibres de collagène.

11. Brin de suture selon la revendication 8, dans lequel ladite enveloppe comprend moins d'environ 82% de la quantité totale de filament dans ledit brin de suture.

12. Brin de suture selon la revendication 8, comprenant en outre un revêtement disposé sur ladite enveloppe.

13. Brin de suture selon la revendication 12, dans lequel ledit revêtement est choisi dans le groupe constitué de cire, de silicone, de caoutchoucs de silicone, de PTFE, de PBA et d'éthycellulose.

14. Brin de suture selon la revendication 1, dans lequel une demi-longueur dudit brin de suture est prévue avec une trace identifiable et l'autre demi-longueur dudit brin de suture ne contient pas de trace identifiable.

15. Ensemble de suture comprenant:
un brin de suture selon l'une quelconque des revendications précédentes; et
un ancrage de suture attaché audit brin de suture.

16. Ensemble de suture comprenant:
un brin de suture selon l'une quelconque des revendications 1 à 14; et
une aiguille demi-ronde, effilée attachée à une extrémité ou les deux dudit brin de suture.
